# EUROPÄISCHE PATENTSCHRIFT

(11) **EP 0 683 661 B1**
(45) Veröffentlichungstag und Bekanntmachung des Hinweises auf die Patenterteilung: **02.04.1997**
(21) Anmeldenummer: 94904946.4
(22) Anmeldetag: 20.01.1994
(51) Int. Cl.: A61K 7/42

(54) **KOSMETISCHE UND DERMATOLOGISCHE LICHTSCHUTZFORMULIERUNGEN MIT EINEM GEHALT AN ANORGANISCHEN MIKROPIGMENTEN**
COSMETIC AND DERMATOLOGICAL PHOTOPROTECTIVE FORMULATIONS CONTAINING INORGANIC MICRO-PIGMENTS
FORMULATIONS COSMETIQUES ET DERMATOLOGIQUES DE PROTECTION SOLAIRE CONTENANT DES MICROPIGMENTS INORGANIQUES

(30) Priorität: 11.02.1993 DE 4303983
(43) Veröffentlichungstag der Anmeldung: 29.11.1995
(73) Patentinhaber: Beiersdorf Aktiengesellschaft, D-20245 Hamburg (DE)
(72) Erfinder: GERS-BARLAG, Heinrich, D-25493 Kummerfeld (DE); SCHULZ, Sabine, D-22529 Hamburg (DE)
(86) Internationale Anmeldenummer: DE9400041
(87) Internationale Veröffentlichungsnummer: WO9417779

(56) Entgegenhaltungen:
- EP-A- 0 518 772
- WO-A-92/21315
- DE-A- 1 492 258
- FR-A- 2 622 440
- GB-A- 2 184 356
- US-A- 4 731 242
- US-A- 4 917 882

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Die anorganischen Pigmente zeichnen sich an sich durch gute Lichtschutzwirkung aus. Sie haben jedoch den Nachteil, daß es schwierig ist, sie in befriedigender Weise solchen Formulierungen einzuverleiben.

Ein weiterer Nachteil des Einsatzes anorganischer Pigmente in kosmetischen Formulierungen ist, daß solche Pigmente in den weitaus meisten Fällen zu starker Hauttrockenheit führen.

Da sinnvollerweise verhindert werden muß, daß sich die Pigmentkörner zu Agglomeraten zusammenballen, mußte den Formulierungen stets ein gewisser Anteil an Emulgatoren oder vergleichbaren ober- oder grenzflächenaktiven Substanzen zugefügt werden.

An sich ist die Verwendung der üblichen kosmetischen Emulgatoren unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, emulgatorfreie Sonnenschutzprodukte zu entwickeln.

Emulgatorfreie Lichtschutzpräparate auf Basis sogenannter Hydrodispersionen sind seit einiger Zeit für den Verbraucher zugängig.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen metastabile Systeme dar, und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Obwohl dieser Formulierungstyp gegenüber den herkömmlichen Lichtschutzformulierungen den Vorteil der Emulgatorfreiheit bietet, gibt es andererseits auch einige Punkte, welche der Verbesserung bedürfen. So ist für eine gute Lichtschutzwirkung solcher Präparate eine vergleichsweise hohe Konzentration an UV-Filtern notwendig. Darüberhinaus fühlen sich solche Präparate im Vergleich zu Emulsionen und Lichtschutzölen klebrig an.

Eine weitere Aufgabe war daher, Lichtschutzpräparate zur Verfügung zu stellen, welche sich durch eine relativ niedrige Konzentration an UV-Filtern auszeichnen und darüberhinaus ein angenehmes Hautgefühl vermitteln. Eine andere Aufgabe der vorliegenden Erfindung bestand darin, nichtklebrige Lichtschutzpräparate zur Verfügung zu stellen.

Es war überraschend und für den Fachmann nicht vorauszusehen, daß
kosmetische und dermatologische Lichtschutzformulierungen mit einem Gehalt an
(a) anorganischen Mikropigmenten, welche oberflächlich hydrophobisiert sind, als UV-Filtersubstanzen sowie
(b) gegebenenfalls zusätzlichen organischen UV-Filtersubstanzen dadurch gekennzeichnet, daß
(c) die Formulierungen Hydrodispersionen darstellen,
(d) welche aus einer inneren Lipidphase und einer äußeren wäßrigen Phase bestehen, und
(e) welche frei von Emulgatoren sind, und daß
(f) die anorganischen Mikropigmente in die Lipidphase der Hydrodispersionen eingearbeitet sind,
die Lösung dieser Aufgaben darstellen.

Zwar werden in den Schriften EP-A 518 772, FR-A 2 622 440, EP-A 456 458, EP-A 456 459 und EP-A 456 460 Lichtschutzformulierungen auf der Basis von TiO₂-Pigmenten beschrieben. Emulgatorfreie Systeme sind aber auf solche Weise nicht realisierbar. Auch sind pigmenthaltige Hydrodispersionen der erfindungsgemäßen Art bisher nicht bekannt gewesen. Auch in den Schriften WO92/21315 und DE-A 14 92 258 werden pigmenthaltige Zubereitungen beschrieben.

Überraschend und nicht vorhersehbar war, daß bei Befolgung der hiermit offenbarten Lehre zum technischen Handeln in jeglicher Hinsicht überaus befriedigende Präparate erhältlich sind. Vielmehr war zu erwarten gewesen, daß sich die Mikropigmentpartikel, mangels eines Emulgators, zu Agglomeraten zusammenballen würden.

Ferner war überraschend, daß die Klebrigkeit der Hydrodispersionen bei Befolgung der hiermit offenbarten Lehre zum technischen Handeln drastisch vermindert werden kann.

Schließlich war überraschend, daß der Einsatz von anorganischen Pigmenten in den erfindungsgemäßen Formulierungen nicht zu starker Hauttrockenheit führt, sondern im Gegenteil ein anhaltendes, äußerst angenehmes Hautgefühl verursacht.

Erfindungsgemäße kosmetische und dermatologische Zubereitungen enthalten bevorzugt anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Voraussetzung für die Verwendbarkeit anorganischer Pigmente für die erfindungsgemäßen Zwecke ist natürlich die kosmetische bzw. dermatologische Unbedenklichkeit der zugrundeliegenden Substanzen.

Im wesentlichen unerheblich für die vorliegende Erfindung ist dabei, in welchen Modifikationen solche Metalloxide vorliegen. TiO₂ beispielsweise kommt in der Natur in drei Hauptmodifikationen (Rutil, Anatas und Brookit) vor, welche grundsätzlich alle gleichermaßen geeignet sind. Ähnliches gilt für die Modifikationen der Eisenoxide usw.

Vorteilhaft ist, den Partikeldurchmesser der verwendeten Pigmente kleiner als 100 nm zu wählen.

Es ist besonders vorteilhaft im Sinne der vorliegenden Erfindung, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß
n TiO₂ + m (RO)₃Si-R' -> n TiO₂ (oberfl.)
erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen MT 100 T von der Firma TAYCA erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorzugsweise enthalten diese zusätzlich mindestens einen UVA-Filter und/oder mindestens einen weiteren UVB-Filter und/oder mindestens ein anorganisches Pigment.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die Lipidphase kann vorteilhaft gewählt werden aus folgender Substanzgruppe:
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Besonders bevorzugt ist, die Komponenten der Lipidphase aus der Gruppe der Silikonöle zu wählen.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten anorganische Pigmente z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den anorganischen Pigmenten öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Vorteilhafte öllösliche UVB-Filtersubstanzen sind z.B.:
- 3-Benzylidencampher-Derivate, vorzugsweise
   3-(4-Methylbenzyliden)campher, 3-Benzylidencampher;
- 4-Aminobenzoesäure-Derivate, vorzugsweise
   4-(Dimethylamino)-benzoesäure(2-ethylhexyl)ester.
   4-(Dimethylamino)benzoesäureamylester;
- Ester der Zimtsäure, vorzugsweise
   4-Methoxyzimtsäure(2-ethylhexyl)ester,
   4-Methoxyzimtsäureisopentylester;
- Ester der Salicylsäure, vorzugsweise
   Salicylsäure(2-ethylhexyl)ester,
   Salicylsäure(4-isopropylbenzyl)ester,
   Salicylsäurehomomenthylester;
- Derivate des Benzophenons, vorzugsweise
   2-Hydroxy-4-methoxybenzophenon,
   2-Hydroxy-4-methoxy-4'-methylbenzophenon,
   2,2'-Dihydroxy-4-methoxybenzophenon;
- Ester der Benzalmalonsäure, vorzugsweise
   4-Methoxybenzalmalonsäuredi(2-ethylhexyl)ester;
- 2,4,6-Trianilino-(p-carbo-2'-ethyl-1'-hexyloxy)-1,3,5-triazin.

Vorteilhafte wasserlösliche UVB-Filtersubstanzen sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise
   2-Hydroxy-4-methoxybenzophenon-5-sulfonsäure und deren Salze;
- Sulfonsäure-Derivate des 3-Benzylidencamphers, wie z.B.
   4-(2-Oxo-3-bornylidenmethyl)benzolsulfonsäure,
   2-Methyl-5-(2-oxo-3-bornylidenmethyl)sulfonsäure und deren Salze.

Die Liste der genannten UVB-Filter, die in Kombination mit den erfindungsgemäßen anorganischen Pigmenten verwendet werden können, soll selbstverständlich nicht limitierend sein.

Es kann auch von Vorteil sein, die erfindungsgemäßen anorganischen Pigmente mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen anorganischen Pigmente mit UVA- und UVB-Filtern zu kombinieren.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1** Sonnengel, Lichtschutzfaktor 12 | |
|---|---|
| | Gew.-% |
| Phenyltrimethicon | 4,50 |
| Carbomer 981 | 1,50 |
| Octylmethoxycinnamat | 7,50 |
| Parsol ^{R} 1789 | 3,00 |
| TiO₂, Partikelgröße < 100 nm | 4,50 |
| Ethanol | 9,00 |
| Glycerin | 4,50 |
| Hydroxypropylmethylcellulose | 0,30 |
| EDTA-Lösung (14 %-ig) | 0,75 |
| Trisaminopromethamin | 2,01 |
| Parfum, Konservierungsmittel, Farbstoffe | q.s. |
| Wasser, VES | ad 100,00 |

| **Beispiel 2** Sonnengel, Lichtschutzfaktor 12 | |
|---|---|
| | Gew.-% |
| Octyldodecanol | 4,50 |
| Carbomer 981 | 1,50 |
| Octylmethoxycinnamat | 7,50 |
| Parsol ^{R} 1789 | 3,00 |
| TiO₂, Partikelgröße < 100 nm | 4,50 |
| Ethanol | 9,00 |
| Butylenglycol | 4,50 |
| Hydroxypropylmethylcellulose | 0,30 |
| EDTA-Lösung (14 %-ig) | 0,75 |
| Trisaminopromethamin | 2,01 |
| Parfum, Konservierungsmittel, Farbstoffe | q.s. |
| Wasser, VES | ad 100,00 |

| **Beispiel 3** Sonnengel, Lichtschutzfaktor 12 | |
|---|---|
| | Gew.-% |
| Ricinusöl | 4,50 |
| Carbomer 981 | 1,50 |
| Octylmethoxycinnamat | 7,50 |
| Parsol ^{R} 1789 | 3,00 |
| TiO₂, Partikelgröße < 100 nm | 4,50 |
| Ethanol | 9,00 |
| Butylenglycol | 4,50 |
| Hydroxypropylmethylcellulose | 0,30 |
| EDTA-Lösung (14 %-ig) | 0,75 |
| Trisaminopromethamin | 2,01 |
| Parfum, Konservierungsmittel, Farbstoffe | q.s. |
| Wasser, VES | ad 100,00 |

## Patentansprüche

1. Kosmetische und dermatologische Lichtschutzformulierungen mit einem Gehalt an
(a) anorganischen Mikropigmenten, welche oberflächlich hydrophobisiert sind, als UV-Filtersinbstanzen sowie
(b) gegebenenfalls zusätzlichen organischen UV-Filtersubstanzen
dadurch gekennzeichnet, daß
(c) die Formulierungen Hydrodispersionen,
(d) welche aus einer inneren Lipidphase und einer äußeren wäßrigen Phase bestehen, und
(e) welche frei von Emulgatoren sind, darstellen und
(f) die anorganischen Mikropigmente in die Lipidphase der Hydrodispersionen eingearbeitet sind.

2. Lichtschutzformulierungen nach Anspruch 1, dadurch gekennzeichnet, daß die anorganischen Pigmente auf den Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle bzw. Abmischungen aus solchen Oxiden basieren.

3. Lichtschutzformulierungen nach Anspruch 1, dadurch gekennzeichnet, daß die wäßrige Phase der Hydrodispersionen ein Verdickungsmittel enthält.

4. Lichtschutzformulierungen nach Anspruch 1, dadurch gekennzeichnet, daß sie 0,01 Gew.-% bis 30 Gew.-%, insbesondere 0,1 Gew.-% bis 6 Gew.-%, bezogen auf das Gesamtgewicht der Formulierungen, an anorganischen Pigmenten enthalten.

5. Verwendung von anorganischen Mikropigmenten, welche oberflächlich hydrophobisiert sind, als UV-Filtersubstanzen sowie gegebenenfalls zusätzlichen organischen UV-Filtersubstanzen zur Herstellung von kosmetischen Lichtschutzformulierungen zum Schutze der Haut vor schädlicher Wirkung von ultraviolettem Lichte, dadurch gekennzeichnet, daß die Formulierungen Hydrodispersionen, welche aus einer inneren Lipidphase und einer äußeren wäßrigen Phase bestehen, und welche frei von Emulgatoren sind, darstellen und daß die anorganischen Mikropigmente in die Lipidphase der Hydrodispersionen eingearbeitet sind.

## Claims

1. Cosmetic and dermatological sunscreen formulations containing
(a) inorganic micropigments, which are hydrophobized on the surface, as UV filter substances and
(b) optionally additional organic UV filter substances
characterized in that
(c) the formulations are hydrodispersions
(d) which consist of an inner lipid and an outer aqueous phase, and
(e) which are free of emulsifiers, and in that
(f) the inorganic micropigments are incorporated in the lipid phase of the hydrodispersions.

2. Sunscreen formulations according to Claim 1, characterized in that the inorganic pigments are based on the metal oxides and/or other poorly water-soluble or -insoluble metal compounds, in particular of the oxides of titanium (TiO₂), zinc (ZnO), iron (e.g. Fe₂O₃), zirconium (ZrO₂), silicon (SiO₂), manganese (e.g. MnO), aluminium (Al₂O₃), cerium (e.g. Ce₂O₃), mixed oxides of the corresponding metals and mixtures of such oxides.

3. Sunscreen formulations according to Claim 1, characterized in that the aqueous phase of the hydrodispersions contains a thickener.

4. Sunscreen formulations according to Claim 1, characterized in that they contain 0.01% by weight to 30% by weight, in particular 0.1% by weight to 6% by weight, based on the total weight of the formulations, of inorganic pigments.

5. Use of inorganic micropigments, which are hydrophobized on the surface, as UV filter substances and optionally additional organic UV filter substances for the production of cosmetic sunscreen formulations for the protection of the skin from the harmful action of ultraviolet light, characterized in that the formulations are hydrodispersions which consist of an inner lipid phase and an outer aqueous phase and which are free of emulsifiers and in that the inorganic micropigments are incorporated into the lipid phase of the hydrodispersions.

## Revendications

1. Formulations photoprotectrices cosmétiques et dermatologiques contenant
(a) des micropigments minéraux qui sont rendus hydrophobes en surface, en tant que substances filtrantes UV, ainsi que
(b) éventuellement des substances filtrantes UV organiques supplémentaires, caractérisées en ce que
(c) les compositions représentent des hydrodispersions
(d) qui consistent en une phase lipidique interne et une phase aqueuse externe, et
(e) qui sont exemptes d'émulsifiants, et en ce que
(f) les micropigments minéraux sont incorporés dans la phase lipidique des hydrodispersions.

2. Formulations photoprotectrices selon la revendication 1, caractérisées en ce que les pigments minéraux sont à base des oxydes métalliques et/ou d'autres composés métalliques peu solubles ou insolubles dans l'eau, en particulier des oxydes du titane (TiO₂), du zinc (ZnO), du fer (par exemple Fe₂O₃), du zirconium (ZrO₂), du silicium (SiO₂), du manganèse (par exemple MnO), de l'aluminium (Al₂O₃), du cérium (par exemple Ce₂O₃), des oxydes mixtes des métaux correspondants ou des mélanges de tels oxydes.

3. Formulations photoprotectrices selon la revendication 1, caractérisées en ce que la phase aqueuse des hydrodispersions contient un épaississant.

4. Formulations photoprotectrices selon la revendication 1, caractérisées en ce qu'elles contiennent de 0,01% en poids à 30% en poids, en particulier de 0,1% en poids à 6% en poids, sur la base du poids total des formulations, de pigments minéraux.

5. Utilisation de micropigments minéraux qui sont rendus hydrophobes en surface, en tant que substances filtrantes UV, ainsi qu'éventuellement de substances filtrantes UV organiques supplémentaires, pour la préparation de formulations photoprotectrices cosmétiques pour la protection de la peau contre l'effet nocif de la lumière ultraviolette, caractérisée en ce que les formulations représentent des hydrodispersions qui consistent en une phase lipidique interne et une phase aqueuse externe, et qui sont exemptes d'émulsifiants, et en ce que les micropigments minéraux sont incorporés dans la phase lipidique des hydrodispersions.
